# EUROPEAN PATENT SPECIFICATION

(11) **EP 0 677 054 B1**
(45) Date of publication and mention of the grant of the patent: **01.04.1998**
(21) Application number: 94905061.1
(22) Date of filing: 12.01.1994
(51) Int. Cl.: C07H 19/01, C07H 17/08, A61K 31/70

(54) **NEW ANTIPARASITIC AGENTS RELATED TO THE MILBEMYCINS AND AVERMECTINS**
NEUE ANTIPARASITISCHE MITTEL, DERIVATE VON MILBEMYCIN UND AVERMECTIN
NOUVEAUX AGENTS ANTIPARASITAIRES APPARENTES AUX MILBEMYCINES ET AUX AVERMECTINES

(30) Priority: 18.01.1993 GB 9300883
(43) Date of publication of application: 18.10.1995
(73) Proprietor: PFIZER INC., New York, N.Y. 10017 (US); Pfizer Limited, Sandwich Kent CT13 9NJ (GB)
(72) Inventor: BISHOP, Bernard, Frank, Sandwich Kent CT13 9NJ (GB); PACEY, Michael, Stephen, Sandwich Kent CT13 9NJ (GB); PERRY, David, Austen, Groton, CT 06340 (US)
(74) Representative: Moore, James William, Dr.
(86) International application number: EP9400095
(87) International publication number: WO9415944

(56) References cited:
- EP-A- 0 379 341
- EP-A- 0 411 897
- EP-A- 0 428 285
- EP-A- 0 428 286
- EP-A- 0 480 693
- EP-A- 0 506 331
- EP-A- 0 519 731
- EP-A- 0 535 734

## Description

This invention relates to new antiparasitic agents, related to the milbemycins and avermectins and to processes for their preparation and compositions thereof.

The avermectins are a group of broad spectrum antiparasitic agents referred to previously as the C-076 compounds. They are produced by fermenting a strain of the micro-organism Streptomyces avermitilis under aerobic conditions in an aqueous nutrient medium containing inorganic salts and assimilable sources of carbon and nitrogen. The isolation and the chemical structure of the eight individual components which make up the C-076 complex is described in detail in British Patent Specification 1573955.

The C-076 complex comprises eight distinct but closely related compounds described as C-076 A1a, A1b, A2a, A2b, B1a, B1b, B2a and B2b. The "a" series of compounds refers to the natural avermectins wherein the 25-substituent is (S)-sec-butyl and the "b" series to those wherein the 25-substituent is isopropyl. The designations "A" and "B" refer to avermectins wherein the 5-substituent is methoxy or hydroxy, respectively, and the numeral "1" refers to avermectins wherein a double bond is present at the 22-23 position, and numeral "2" to avermectins lacking the 22-23 double bond and having a hydrogen at the 22-position and hydroxy at the 23 position.

In our European Patent Applications 0214731, 0284176, 0317148, 0308145, 0340832, 0335541 and 0350187 there are described preparations of compounds related to the avermectins but having a group at the 25-position other than the isopropyl or (S)-sec-butyl groups found in the original avermectin compounds disclosed in British Patent Specification 1573955. Such compounds may be prepared by fermentation of particular strains of Streptomyces avermitilis in the presence of organic acids or derivatives thereof. Production of such avermectins is described in Journal of Antibiotics (1991), 44, No. 3, pp 357-365.

The milbemycins form another group of related macrolides which are distinguished from the avermectins in lacking a sugar residue attached at the C-13 position. Examples of such compounds are described in UK patent 1390336, and European patent publications 170006, 254583, 334484 and 410615. In addition to these fermentation products a large number of publications describe compounds derived semisynthetically from these fermentation products many of which possess useful antiparasitic activities. Some of this chemistry is reviewed in Macrolide Antibiotics, Omura S., Ed., Academic press, New York (1984) and by Davies, H.G., Green, R.H. in Natural product Reports (1986), 3, 87-121 and in Chem. Soc. Rev., 1991, 20, 271-339.

Thus for example EP-A-0519731 discloses 4a-substituted avermectin derivatives; EP-A-0506331 and EP-A-0411897 disclose 4' and 4"-substituted avermectin derivatives and EP-A-0428286 and EP-A-0480693 disclose avermectin derivatives modified at the C-24 and C-25 positions. EP-A-0379341 and EP-0110667 disclose avermectin and milbemycin derivatives respectively having a 5-oxime substituent however none of these prior art patents exemplify avermectin monosaccharides having a 5-oxime substituent.

It has been found that certain compounds synthetically derivable from known avermectins and avermectin derivatives possess unexpected beneficial biological properties.

According to one aspect of the invention there are provided compounds of formula (I): 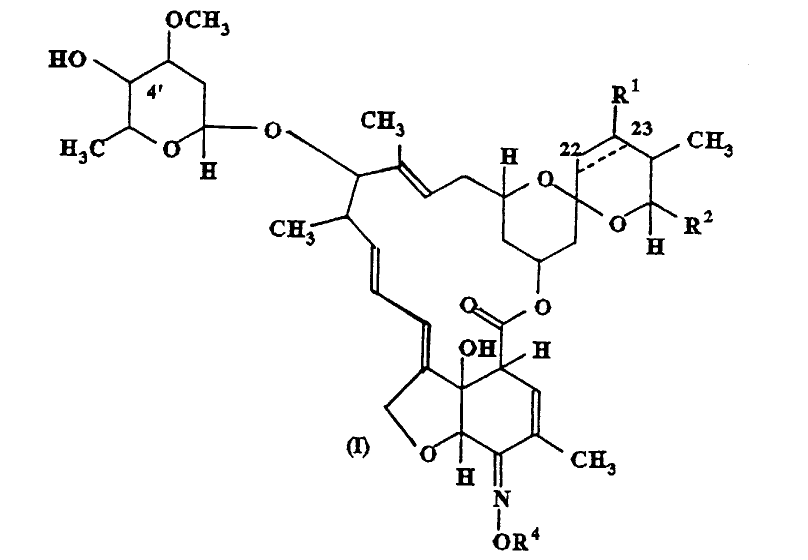 wherein the broken line at the 22-23 position represents an optional bond and either this bond is present and
R¹ is absent or this bond is absent and R¹ is H or OH;
R² is a C₁-C₈ alkyl, or C₃-C₈ cycloalkyl group; and
R⁴ is H or a group capable of being hydrolysed in vivo to yield a compound in which R⁴ is H.

Unless the context otherwise requires, all alkyl and alkenyl substituents having 3 or more carbon atoms may be straight or branched-chain. The term "aryl" includes phenyl which may be substituted by at least one C₁-C₆ alkyl, hydroxy, C₁-C₆ alkoxy, halo, nitro or CF₃ group. In the present invention the term "alkyl" is intended to indicate those alkyl groups of from 1 to 8 carbon atoms such as methyl, ethyl, propyl, isopropyl, butyl, pentyl, hexyl, and the like, either straight or branched chain. The term "alkanoyl" is intended to include those alkanoyl groups of from 1 to 8 carbon atoms such as formyl, acetyl, propionyl, buytryl, pentanoyl, hexanoyl, and the like.

The term "carbamoyl" is intended to indicate the group -CONR₇R₈ where R₇ and R₈ are the same or different and are H, alkyl, aryl, heteroaryl or form a 4-8 membered ring containing 1 or more O, N, or S atoms.

Groups hydrolysable in vivo to yield corresponding compounds in which the group is replaced by H are well known in the pharmaceutical art in general and a wide variety of such groups are suitable for use in the compounds of this invention. Examples of such groups are C₂-C₈ alkanoyl, aroyl, carbamoyl, C₁-C₈ alkoxycarbonyl groups, and dicarboxylic acid and amino acid residues. Particular groups are identified in the Examples below. Preferred compounds are those in which R² is cyclohexyl,
and the optional bond at the 22-23 position is absent and R¹ is H.

The oxime monosaccharides in which R⁴ is H are particularly preferred.

Individual compounds within the invention are described in the Examples given below.

The most preferred compound is 5-oximino-22,23-dihydro-25-cyclohexyl avermectin B1 monosaccharide.

According to another aspect of the invention, there is provided a process for preparing such a compound which comprises the steps (1) of oxidising a compound of formula (II): 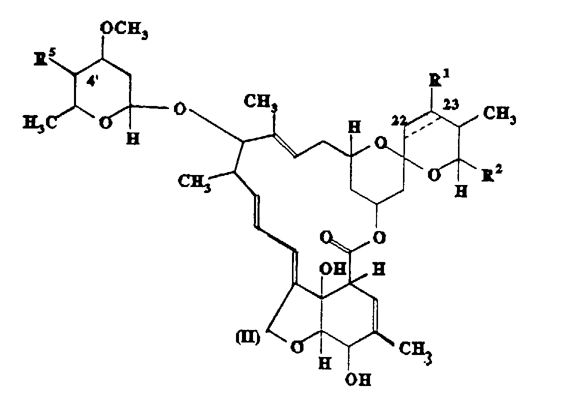 wherein the broken line, R¹ and R² are as defined above and R⁵ is OH or R⁵ is ∝-oleandrosyloxy to yield a compound of formula (III) : 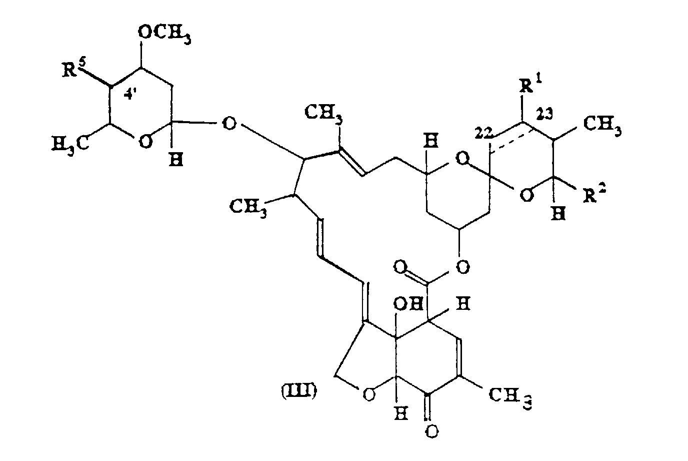
and (ii) allowing the compound of formula (III) to react with a compound of formula R⁴-O-NH₂ where R⁴ is as defined above and where R⁵ is α-oleandrosyloxy, hydrolysing the compound obtained to yield a compound of formula (I),
and (iii) if necessary replacing group R⁴ when the latter is H with said group capable of being hydrolysed in vivo to yield a compound in which R⁴ is H, and
   if necessary,
(iv) hydrogenating the compound to reduce the double bond at the 22-23 position to a single bond.
Preparation of compounds according to the invention is discussed, by way of illustration, below.

The compounds of the present invention may be prepared starting from the compounds of formula (iv), which may themselves be prepared as described in the above-mentioned patent publications. 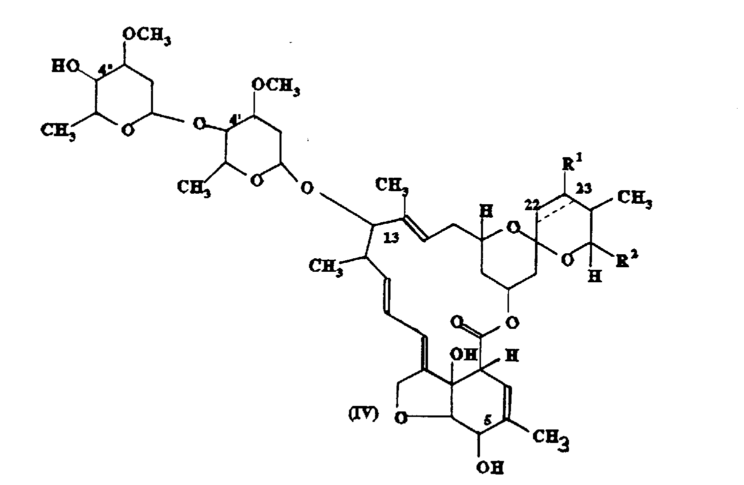
Compound IIa Double bond present, R¹ absent.
Compound IIb Double bond absent, R¹ = H.
Compound IIc Double bond absent, R¹ = OH.

The semisynthetic modifications required to provide the compounds given by formula I may require sequential reactions and the exact order in which these transformations are performed may vary.
Compounds having a 23-hydroxyl group (or a protected derivative) may be converted to either the corresponding 22,23-dihydro compound or alternatively to the corresponding compound having a double bond at the 22-23 position using methods described in US patent 4328335. The latter compounds may also be hydrogenated to the 22,23-dihydro compounds using Wilkinson's catalyst under conditions described in US patent 4199569.

The preparation of the compounds of the invention can be accomplished by first converting the aforementioned disaccharides IIa, b and c into their corresponding monosaccharides by hydrolysis. An alternative method of preparing the monosaccharides comprises a direct fermentation procedure starting from a corresponding aglycone as described in European Patent Application 463677.

Alternatively, the compounds of the invention may be prepared by carrying out the above synthetic manipulations on the disaccharides IIa, b or c and finally hydrolysing them to the desired monosaccharides.

When desired, hydroxy groups may be acylated to give esters using reagent such as acid anhydrides or acid chlorides and amine bases according to general procedures known to those skilled in the art. Hydroxy groups may be converted to oxo groups by oxidation with manganese dioxide or tetrapropylammonium perruthenate. The oxo compound may be treated with hydroxylamine or an O-substituted analogue thereof to produce the corresponding oxime.

The compounds of the invention are effective in treating a variety of conditions caused by endoparasites including, in particular, helminthiasis which is most frequently caused by a group of parasitic worms described as nematodes and which can cause severe economic losses in swine, sheep, horses and cattle as well as affecting domestic animals and poultry. The compounds are also effective against other nematodes which affect various species of animals including, for example:- Dirofilaria in dogs and various parasites which can infest livestock, companion animals such as cats and dogs and also humans including gastro-intestinal parasites such as Ancylostoma, Necator, Ascaris, Strongyloides, Trichinella, Capillaria, Toxocara, Toxascaris, Trichuris, Enterobius and parasites which are found in the blood or other tissues and organs such as filarial worms and the extra intestinal stages of Strongyloides, Toxocara and Trichinella.

The compounds are also of particular value in treating ectoparasite infections including particular arthropod ectoparasites of humans, animals and birds such as ticks, mites, lice, fleas, blowfly, biting insects and migrating dipterous larvae which can affect cattle and horses.

The compounds are also insecticides active against household pests such as the cockroach, clothes moth, carpet beetle and the housefly as well as being useful against arthropod pests of stored grain and of agricultural plants such as spider mites, aphids, caterpillars and against migratory orthopterans such as locusts. We have discovered that compounds within the scope of this invention are both safe and have unexpectedly high potent systemic activity against fleas and other important arthropod parasites of cats and dogs.

The compounds of formula (I) may be administered as a formulation appropriate to the specific use envisaged and to the particular species of host animal being treated and the parasite or insect involved. The compounds may be administered by injection, either subcutaneously or intramuscularly, alternatively they may be administered orally in the form of a capsule, bolus, tablet, chewable tablet or liquid drench, or they may be administered as a topical formulation or as an implant. For topical application dip, spray, powder, dust, pour-on, spot-on, jetting fluid, shampoos, collar, tag or harness may be used. Such formulations are prepared in a conventional manner in accordance with standard veterinary practice. Thus capsules, boluses or tablets may be prepared by mixing the active ingredient with a suitable finely divided diluent or carrier, additionally containing a disintegrating agent and/or binder such as starch, lactose, talc, or magnesium stearate. A drench formulation may be prepared by dispersing the active ingredient in an aqueous solution together with dispersing or wetting agents and injectable formulations may be prepared in the form of a sterile solution or emulsion. Pour-on or spot-on formulations may be prepared by dissolving the active ingredient in an acceptable liquid carrier vehicle, such as butyl digol, liquid paraffin or non-volatile ester with or without addition of a volatile component such as isopropanol. Alternatively, pour-on, spot-on or spray formulations can be prepared by encapsulation to leave a residue of active agent on the surface of the animal. These formulations will vary with regard to the weight of active compound depending on the species of host animal to be treated, the severity and type of infection and the body weight of the host. The compounds may be administered continuously, particularly for prophylaxis, by known methods. Generally for oral, parenteral and pour-on administration a dose of from about 0.001 to 10mg per kg of animal body weight given as a single dose or in divided doses for a period of from 1 to 5 days will be satisfactory but of course there can be instances where higher or lower dosage ranges are indicated and such are within the scope of this invention.

As an alternative the compounds may be administered with the animal feedstuff and for this purpose a concentrated feed additive or premix may be prepared for mixing with the normal animal feed.

For use as an insecticide and for treating agricultural pests the compounds are applied as sprays, dusts, pour-on formulations, emulsions and the like in accordance with standard agricultural practice.

For human use the compounds are administered as a pharmaceutically acceptable formulation in accordance with normal medical practice.

The preparation of compounds according to the invention are illustrated by the following Examples.

Examples 13 to 24 are examples of compounds in which R⁴ is a group hydrolysable in vivo.

### EXAMPLE 1

### 22,23-Dihydroavermectin B1a monosaccharide

22,23-Dihydroavermectin B1a (50g) was dissolved in a mixture of isopropanol (100ml) and sulphuric acid (1ml) and stirred at room temperature under a nitrogen atmosphere for 48 hours. The reaction mixture was poured onto crushed ice and extracted with dichloromethane (2 x 200ml). The combined extracts were washed with aqueous saturated sodium bicarbonate solution (100ml), dried over anhydrous magnesium sulphate and concentrated under vacuum to give white crystals (14g) which were removed by filtration. Mass and NMR spectra were fully consistent with the proposed structure.
The B1b analogue was obtained by an identical method starting from 22,23-dihydroavermectin B1b.

### EXAMPLE 2

### 5-Oxo-22,23-dihydroavermectin B1a monosaccharide

22,23-dihydroavermectin B1a monosaccharide (14g) was dissolved in diethyl ether (200ml) and activated manganese dioxide (14g) added. The mixture was stirred at room temperature for 4 hours, filtered and evaporated to dryness under vacuum to yield the title product (11.4g) whose NMR spectrum was fully consistent with the proposed structure.
The B1b analogue was obtained by an identical method starting from 22,23-dihydroavermectin B1b monosaccharide.

### EXAMPLE 3

### 5-Oximino-22,23-dihydroavermectin B1a monosaccharide

5-Oxo-22,23-dihydroavermectin B1a monosaccharide (1g) was dissolved in dry pyridine (25ml) and hydroxylamine hydrochloride (1g) added. The stirred reaction mixture was heated under reflux for 4 hours and after cooling poured onto crushed ice and extracted with dichloromethane (2 x 50ml). The combined extracts were dried over anhydrous magnesium sulphate and evaporated under vacuum to give a crude gum (1.1g). This material was purified using high pressure liquid chromatography on a Dynamax (trade mark) column (41.4 x 250mm, 8µm, ODS-silica, Rainin) eluting with methanol - water 83:17 at 42ml per minute. Appropriate fractions were combined and evaporated to dryness to yield the title product as a white solid, melting point 180-190°C. Mass and NMR spectra were fully consistent with the proposed structure.
5-Oximino-22,23-dihydroavermectin B1b was prepared by an identical method starting from 5-oxo-22,23-dihydroavermectin B1b monosaccharide.

### EXAMPLE 4

### 22,23-Dihydro-25-cyclohexylavermectin B1 monosaccharide

25-Cyclohexylavermectin B1 (9.9g) was dissolved in toluene (1 litre) and Wilkinson's catalyst (tris(triphenylphosphine)rhodium(I) chloride) (9.25g) was added. The solution was hydrogenated on a large Parr (trade mark) shaker at room temperature at 3.45 bar(50 psi) hydrogen pressure. After 3 hours the reaction vessel was depressurised and allowed to stand for 12 hours before addition of a further portion of catalyst (5g) and hydrogenated as before for a further 2 hours after which no starting material remained. The solution was filtered, evaporated to dryness under vacuum and the residue chromatographed on silica eluting with dichloromethane then dichloromethane:methanol 9:1. The crude product was then chromatographed again on silica (200g) eluting with dichloromethane:methanol 19:1 to give after evaporation of the solvent under vacuum impure 22,23-dihydro-25-cyclohexylavermectin B1 as a brown foam (10g). This material was dissolved in a mixture of isopropanol (200ml) and sulphuric acid (2ml) and the brown solution was stirred at room temperature for 15 hours then poured into a mixture of ice and water (500ml) and extracted with dichloromethane (3 x 200ml). The organic layer was washed with saturated aqueous potassium hydrogen carbonate solution (100ml), water (2 x 50ml) dried over anhydrous magnesium sulphate and evaporated under vacuum to give a crude gum which was chromatographed on silica (100g) eluting with dichloromethane then dichloromethane:ethyl acetate 2:1 to give the title compound (8.2g). Mass and NMR spectra were fully consistent with the proposed structure.

### EXAMPLE 5

### 5-Oximino 22,23-dihydro-25-cyclohexylavermectin B1 monosaccharide

22,23-Dihydro-25-cyclohexylavermectin B1 monosaccharide (8.2g) was oxidized to the 5-oxo derivative using manganese dioxide in anhydrous diethyl ether according to the procedure of Example 2. The crude product was purified by chromatography on silica (50g) to give the 5-oxo compound (3.22g) as a yellow foam. This was dissolved in anhydrous pyridine (60ml) and hydroxylamine hydrochloride (3.22g) was added. After stirring for 15 hours at room temperature a further aliquot of hydroxylamine hydrochloride (3.22g) was added and the solution heated to 50°C until no starting material remained. The solution was poured into water (50ml) and extracted with diethyl ether (3 x 50ml). The organic layer was washed with water, saturated sodium chloride solution, dried over anhydrous sodium sulphate and evaporated to dryness under vacuum. The crude product was chromatographed on silica (25g) eluting with dichloromethane: ethyl acetate 4:1 and finally purified by high pressure liquid chromatography using a Dynamax (trade mark) column (41.4 x 250mm, 8µm ODS-silica, Rainin) eluting with methanol:water 9:1 at 65ml per minute. Appropriate fractions were combined and evaporated under vacuum to give the title compound (1.53g). Mass and NMR spectra were fully consistent with the proposed structure.

### EXAMPLE 6

### 25-Cyclohexylavermectin B2 monosaccharide

25-Cyclohexylavermectin B2 (10g) was suspended in isopropanol (100ml) and a solution of sulphuric acid (2ml) in isopropanol (100ml) was added. After stirring at room temperature for 24 hours the clear solution was poured into ice (600g) and extracted with dichloromethane (2 x 100ml). The organic layer was dried over anhydrous sodium sulphate and evaporated to dryness. The residue was dissolved in tetrachloromethane and the solution stored at 4°C. The crystals which separated slowly were periodically removed by filtration and found to be the pure title compound. Mass and NMR spectra were fully consistent with the proposed structure.

### EXAMPLE 7

### 5-Oximino-25-cyclohexylavermectin B2 monosaccharide

Using the procedures of Examples 2 and 3, 25-cyclohexylavermectin B2 monosaccharide was converted to the title compound. Mass and NMR spectra were fully consistent with the proposed structure.

### EXAMPLE 8

### 25-Cyclohexylavermectin B1 monosaccharide

25-Cyclohexylavermectin B1 (20g) was dissolved in tetrahydrofuran (250ml) and a mixture of tetrahydrofuran (250ml), water (10ml) and sulphuric acid (10ml) was added. The mixture was stirred at room temperature for 15 hours then poured into a mixture of ice (500g) and water (1 l) and extracted with dichloromethane (2 x 500ml). The organic layer was washed with saturated aqueous sodium chloride solution, dried over anhydrous sodium sulphate and evaporated under vacuum to give a foam. This was chromatographed on silica (150g) eluting with ethyl acetate - dichloromethane 1:1 to give a crude product (13.3g). Final purification was achieved by reverse phase hplc using a Dynamax (trade mark) column (41.4 x 250mm, 8µm ODS-silica, Rainin) eluting with methanol-water 4:1 at 70ml per minute to give the pure title compound. Mass and NMR spectra were fully consistent with the proposed structure.

### EXAMPLE 9

### 5-Oximino-25-cyclohexylavermectin B1 monosaccharide

Using the procedures of Examples 2 and 3, 25-cyclohexylavermectin B1 monosaccharide was converted to the title compound. Mass and NMR spectra were fully consistent with the proposed structure.

### EXAMPLE 10

### 5-Oxo-avermectin B1a

Avermectin B1a (2.4g) was dissolved in diethyl ether (50ml) and activated manganese dioxide (2.0g) added. The mixture was stirred at room temperature for 18 hours, filtered and evaporated to dryness under vacuum to yield the title product whose NMR spectrum was fully consistent with the proposed structure.

### EXAMPLE 11

### 5-Oximino-avermectin B1a

5-Oxo-avermectin B1a (800mg) (Example 10) was dissolved in pyridine (10ml) and hydroxylamine hydrochloride (800mg) added. After stirring at room temperature for 1 hour the mixture was poured into an ice (50g) and water(50ml) mixture, acidified to pH 4 with concentrated hydrochloric acid and extracted with dichloromethane (3 x 30ml). The combined extracts were washed with water (20ml), dried over anhydrous sodium sulphate and evaporated to dryness under reduced pressure to yield a crude material (1g). This material was chromatographed on silica (Kieselgel 60, 230-400 mesh, Merck) (100g) eluting with dichloromethane: ethyl acetate 2:1 and finally purified by high pressure liquid chromatography using a Dynamax (trade mark) column (41.4 x 250mm, 8µm ODS-silica, Rainin) eluting with methanol:water 85:15 at 70ml per minute. Appropriate fractions were combined and evaporated under vacuum to give the title compound (290mg). Mass and NMR spectra were fully consistent with the proposed structure.

### EXAMPLE 12

### 5-Oximino-avermectin B1a monosaccharide

5-Oximino-avermectin B1a (50mg) (Example 11) was dissolved in a mixture of isopropanol (1 ml) and sulphuric acid (10 µl) and stirred at room temperature under a nitrogen atmosphere for 48 hours. A saturated aqueous solution of sodium bicarbonate (1 ml) was then added and the product extracted with ethylacetate (2 x 5 ml). The combined extracts were dried over anhydrous magnesium sulphate and concentrated under vacuum. The resulting crude product (25 mg) was purified using high pressure liquid chromatography on an Ultrasphere (trade mark) column (24 x 250 mm, 5 microns, ODS-silica, Beckman) eluting with methanol: water 85:15 at 20 ml per minute. Appropriate fractions were combined, to give the title product. Mass and NMR spectra were fully consistent with the proposed structure.

### EXAMPLE 13

### 5-(Trimethylacetyloximino)-25-cyclohexyl-22,23-dihydroavermectin B1 monosaccharide

To a stirred solution of 5-oximino-25-cyclohexyl-22,23-dihydroavermectin B1 monosaccharide (Example 5) (50mg) in dichloromethane (2ml) at room temperature was added triethylamine (72µl) followed by trimethylacetyl chloride (80µl). After leaving to stand for 18 hrs an aqueous citric acid solution (10% w/v, 2ml) was added and the organic layer separated, washed with saturated aqueous sodium chloride solution (2ml), dried over anhydrous sodium sulphate and evaporated to dryness under vacuum to give the crude product which was chromatographed on silica (Kieselgel 60, 230-400 mesh,
Merck) (5g) eluting with diethyl ether.
Appropriatefractions were combined and evaporated to dryness under vacuum to give a material (53mg) which was further purified by high pressure liquid chromatography on a Dynamax (trade mark) column (21.2 x 250mm, 5µm, ODS-silica, Rainin) eluting at 20ml per minute with a methanol - water 95:5 mixture. Appropriate fractions were combined and evaporated under vacuum to yield the title compound as a white powder (18mg). Mass and NMR spectra were fully consistent with the proposed structure.

### EXAMPLE 14

### 5-(Benzoyloximino)-25-cyclohexyl-22,23-dihydroavermectin B1 monosaccharide

5-oximino-25-cyclohexyl-22,23-dihydroavermectin B1 monosaccharide (Example 5) (70mg) in dichloromethane (30ml) was reacted with triethylamine (50µl) and benzoyl chloride (100µl) and the desired product extracted in a manner identical to that described in Example 13. Purification was achieved by high pressure liquid chromatography on a Dynamax (trade mark) column (41.4 x 250mm, 8µm, ODS-silica, Rainin) eluting at 45ml per minute with a methanol - water 90:10 mixture. Appropriate fractions were combined and evaporated under vacuum to yield the title compound as a white powder (28mg). Mass and NMR spectra were fully consistent with the proposed structure.

### EXAMPLE 15

### 5-(N-Methylcarbamoyloximino)-25-cyclohexyl-22,23-dihydroavermectin B1 monosaccharide

To a stirred solution of 5-oximino-25-cyclohexyl-22,23-dihydroavermectin B1 monosaccharide (Example 5) (106mg) in dichloromethane (10ml) was added methyl isocyanate (15µl) and the mixture stirred for lhr. A further amount of methyl isocyanate (30µl) was then added and the reaction stirred for another 72 hrs before adding saturated aqueous sodium chloride solution (10ml) and ether (30ml). The organic extract was dried over anhydrous sodium sulphate and evaporated to dryness under vacuum to give the crude product (150mg) which was purified by high pressure liquid chromatography on a Dynamax (trade mark) column (41.4 x 250mm, 8µm, ODS-silica, Rainin) eluting at 45ml per minute with a methanol - water 91:9 mixture. Appropriate fractions were combined and evaporated under vacuum to yield the title compound as a white powder (80mg). Mass and NMR spectra were fully consistent with the proposed structure.

### EXAMPLE 16

### 5-(N,N-Dimethylcarbamoyloximino)-25-cyclohexyl-22,23-dihydroavermectin B1 monosaccharide

To a stirred solution of 5-oximino-25-cyclohexyl-22,23-dihydroavermectin B1 monosaccharide (Example 5) (50mg) in dichloromethane (2ml) at room temperature was added triethylamine (72µl) and 4-dimethylaminopyridine (1mg) followed by N,N-dimethylcarbamoyl chloride (58µl). After 3hrs further N,N-dimethylcarbamoyl chloride (58µl) was added and the reaction left to stand for 18 hrs. An aqueous citric acid solution (10% w/v, 2ml) and diethyl ether (20ml) were then added and the organic layer separated, washed with saturated aqueous sodium chloride solution (5ml), dried over anhydrous sodium sulphate and evaporated to dryness under vacuum to give the crude product which was purified by high pressure liquid chromatography on a Dynamax (trade mark) column (21.2 x 250mm, 5µm, ODS-silica, Rainin) eluting at 10ml per minute with a methanol - water 90:10 mixture. Appropriate fractions were combined and evaporated under vacuum to yield the title compound as a white powder (18mg). Mass and NMR spectra were fully consistent with the proposed structure.

### EXAMPLE 17

### 5-(4-Methylpiperazinyl-1-carbonyloximino)-25-cyclohexyl- 22,23-dihydroavermectin B1 monosaccharide

To a stirred solution of N-methylpiperazine (0.65ml) and triethylamine (1.3ml) in toluene (25ml) at 0°C was added dropwise a solution of phosgene in toluene (20% , 5.1ml) over a period of 15 min. The reaction was allowed to warm to room temperature, stirred for 3hr, filtered and concentrated to approximately 10ml under reduced pressure to give a solution of 1-chlorocarbonyl-4-methylpiperazine which was reacted with 5-oximino-25-cyclohexyl-22,23-dihydroavermectin B1 monosaccharide (Example 5) (300mg), triethylamine (110µl) and 4-dimethylaminopyridine (5mg) in dichloromethane (10ml) at room temperature according to the method described in Example 16. Purification of the desired material was achieved by chromatography on silica (Kieselgel 60, 230-400 mesh, Merck) (35g) eluting with dichloromethane. Appropriate fractions were combined and evaporated to dryness under vacuum to give a material (53mg) which was further purified by high pressure liquid chromatography on a Dynamax (trade mark) column (21.2 x 250mm, 5µm, ODS-silica, Rainin) eluting at 20ml per minute with a methanol - water 95:5 mixture. Appropriate fractions were combined and evaporated under vacuum to yield the title compound as a white powder. Mass and NMR spectra were fully consistent with the proposed structure.

### EXAMPLE 18

### 5-(t-Butyloxycarbonyloximino)-25-cyclohexyl-22,23-dihydroavermectin B1 monosaccharide

To a stirred solution of 5-oximino-25-cyclohexyl-22,23-dihydroavermectin B1 monosaccharide (Example 5) (60mg) and triethylamine (50µl) in dichloromethane (5ml) at room temperature was added t-butyloxycarbonyl anhydride (60mg). After allowing to stand for 48hrs the reaction was evaporated to dryness under vacuum to give a residue which was dissolved in dichloromethane and chromatographed on silica (Kieselgel 60, 230-400 mesh, Merck) (5g) eluting with dichloromethane. Appropriate fractions were combined and evaporated to dryness under vacuum to give the title compound as a white powder (45mg). Mass and NMR spectra were fully consistent with the proposed structure.

### EXAMPLE 19

### 5-(N-(4-Formylphenyl)-carbamoyloximino)-25-cyclohexyl-22, 23-dihydroavermectin B1 monosaccharide

4-Formylphenylisocyanate was prepared according to the method described in J. Med. Chem., 32(10), 2354, (1989) and was reacted with 5-oximino-25-cyclohexyl-22,23-dihydroavermectin B1 monosaccharide (Example 5) (500mg), in dry dichloromethane (50ml) at room temperature for lhr according to the method described in Example 14.
Purification of the desired material was achieved using chromatography over silica (Kieselgel 60, 230-400 mesh, Merck) (125g) eluting with a gradient of hexane - ether 1:1 changing to 20:80. Appropriate fractions were combined and evaporated to dryness under vacuum to give the title compound as a white powder (300mg). Mass and NMR spectra were fully consistent with the proposed structure.

### EXAMPLE 20

### 5-(N-(4-(Diethylaminomethyl)phenyl)-carbonyloximino)-25 -cyclohexyl-22,23-dihydroavermectin B1 monosaccharide

4-(Diethylaminomethyl)benzoyl chloride was prepared according to the method described in United States Patent publication US-4623486 and was reacted with 5-oximino-25-cyclohexyl-22,23-dihydroavermectin B1 monosaccharide (Example 5) (100mg), in dry dichloromethane (50ml) containing triethylamine (450l) and 4-dimethylaminopyridine (126mg) at room temperature for 1hr according to the method described in Example 16. Purification of the desired material was achieved using chromatography over silica (Kieselgel 60, 230-400 mesh, Merck) (5g) eluting with a gradient of methanol - dichloromethane 0:100 changing to 10:90.
Appropriatefractions were combined and evaporated to dryness under vacuum to give the title compound as a white powder (11mg). Mass and NMR spectra were fully consistent with the proposed structure.

### EXAMPLE 21

### 5-(N-(4-(4-Methyl-1-piperazinylmethyl)phenyl)-carbonyloximino)-25-cyclohexyl-22,23-dihydroavermectin B1 monosaccharide

4-(4-Methylpiperazin-1-ylmethyl)benzoyl chloride was prepared according to the method described in United States Patent publication US-4623486 and was reacted with 5-oximino-25-cyclohexyl-22,23-dihydroavermectin B1 monosaccharide (Example 5) in a manner identical to that described in Example 19. The title compound was obtained as a white powder (18mg). Mass and NMR spectra were fully consistent with the proposed structure.

### EXAMPLE 22

### 5-(N-(3-Pyridylcarbonyl)-carbamoyloximino)-25-cyclohexyl- 22,23-dihydroavermectin B1 monosaccharide

To a stirred solution of nicotinamide (4.88g) in dry 1,2-dichloroethane (500ml) was added dropwise oxalyl chloride (5.24ml). The mixture was heated under reflux for 4.5h then cooled, filtered and the resulting solution containing nicotinoyl isocyanate (50ml) was reacted with 5-oximino-25-cyclohexyl-22,23-dihydroavermectin B1 monosaccharide (Example 5) (500mg), in dichloromethane (10ml) at room temperature. After allowing to stand for 18hr further nicotinoyl isocyanate solution (25ml) was added and the mixture left at room temperature for a further 18hr before evaporating to dryness under vacuum to give a residue which was purified by high pressure liquid chromatography on a Dynamax (trade mark) column(41.4 x 250mm, 8µm, ODS-silica, Rainin) eluting at 45ml per minute with a methanol - acetonitrile - water 20:65:15 mixture. Appropriate fractions were combined and evaporated under vacuum to yield the title compound as a white powder. Mass and NMR spectra were fully consistent with the proposed structure.

### EXAMPLE 23

### 5-(N-(3-Pyridyl)-carbamoyloximino)-25-cyclohexyl-22,23-dihydroavermectin B1 monosaccharide

To a solution of nicotinic acid hydrazide dihydrochloride (2g) in water (10ml) was added a solution of sodium nitrite (1.6g) in water (10ml), keeping the temperature below 20°C. Diethyl ether (50ml) was then added and the mixture basified by the careful addition of solid sodium bicarbonate. The organic layer was separated, washed with water (20ml), dried over anhydrous magnesium sulphate and evaporated to dryness under vacuum to give nicotinyl azide (1.1g) m.pt. 54°C. This azide (1.1g) was stirred in dry toluene (10ml) and heated at 100°C under a nitrogen atmosphere for 8hr to give a solution containing 3-pyridyl isocyanate. A portion of this solution (1ml) was reacted with 5-oximino-25-cyclohexyl-22,23-dihydroavermectin B1 monosaccharide (Example 5) (100mg), in toluene (10ml) at room temperature for 1hr before pouring into a diethyl ether - water mixture (1:1, 30ml). The organic layer was separated, dried over anhydrous magesium sulphate and evaporated to dryness under vacuum to give a residue (130mg) which was purified by high pressure liquid chromatography on a Dynamax (trade mark) column (41.4 x 250mm, 8µm, ODS-silica, Rainin) eluting at 45ml per minute with a methanol - water 85:15 mixture, changing to87:13 after 15 mins. Appropriate fractions were combined and evaporated under vacuum to yield the title compound as a white powder (52mg). Mass and NMR spectra were fully consistent with the proposed structure.

### EXAMPLE 24

### 5-(N-Allylcarbamoyloximino)-25-cyclohexyl-22,23-dihydroavermectin B1 monosaccharide

5-oximino-25-cyclohexyl-22,23-dihydroavermectin B1 monosaccharide (Example 5) (500mg) was reacted with allyl isocyanate (108mg) in dichloromethane (50ml) according to the method described in Example 14 to give the title compound as a white powder (352mg). Mass and NMR spectra were fully consistent with the proposed structure.

## Claims

1. A compound of formula (I). 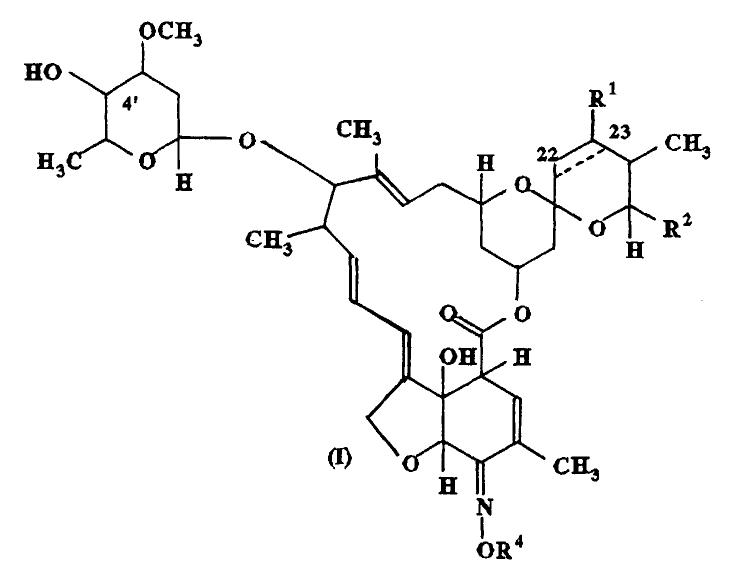 wherein the broken line at the 22-23 position represents an optional bond and either this bond is present and
R¹ is absent or this bond is absent and R¹ is H or OH;
R² is a C₁-C₈ alkyl, or C₃-C₈ cycloalkyl group; and
R⁴ is H or a group capable of being hydrolysed in vivo to yield a compound in which R⁴ is H.

2. A compound according to Claim 1, in which said group capable of being hydrolysed in vivo is an acetyl, t-butylcarbonyl, t-butyloxycarbonyl, benzoyl, methylpiperazinecarbonyl, N-methylcarbamoyl, N,N-dimethylcarbamoyl, N-4-(formylphenyl)carbamoyl, N-(4-diethylaminomethylphenyl)-carbonyl, N-(4-methyl-1-piperazin-methylphenyl)-carbonyl, N-(3-pyridylcarbonyl)-carbamoyl, N-(3-pyridyl)-carbamoyl or N-allylcarbamoyl group.

3. A compound according to Claim 1, in which R² is cyclohexyl.

4. A compound according to any preceding claim, in which the optional bond at the 22-23 position is absent and R¹ is H.

5. Any one of the following compounds:
5-oximino-22,23-dihydroavermectin B1a monosaccharide,
5-oximino-22,23-dihydro-25-cyclohexylavermectin B1 monosaccharide,
5-oximino-25-cyclohexylavermectin B2 monosaccharide,
5-oximino-25-cyclohexylavermectin B1 monosacccharide,
5-oximino-avermectin B1a monosaccharide

6. The compound 5-oximino-22,23-dihydro-25-cyclohexylavermectin B1 monosaccharide.

7. A compound as claimed in claim 1 wherein the group R⁴ is a group hydrolysable in-vivo and said compound is:
5-(trimethylacetyloximino)-25-cyclohexyl-22,23-dihydroavermectin B1 monosaccharide,
5-(benzoyloximino)-25-cyclohexyl-22,23-dihydroavermectin B1 monosaccharide,
5-(N-methylcarbamoyloximino)-25-cyclohexyl-22,23-dihydroavermectin B1 monosaccharide,
5-(N,N-dimethylcarbamoyloximino)-25-cyclohexyl-22,23-dihydroavermectin B1 monosaccharide,
5-(4-methylpiperazinyl-1-carbonyloximino)-25-cyclohexyl-22,23-dihydroavermectin B1 monosaccharide,
5-(t-butyloxycarbonyloximino)-25-cyclohexyl-22,23-dihydroavermectin B1 monosaccharide,
5-(N-(4-formylphenyl)-carbamoyloximino)-25-cyclohexyl-22,23-dihydroavermectin B1 monosaccharide,
5-(N-(4-(diethylaminomethyl)phenyl)-carbonyloximino)-25-cyclohexyl-22,23-dihydroavermectin B1 monosaccharide,
5-(N-(4-(4-methyl-1-piperazinyl-methyl)phenyl)-carbonyloximino)-25-cyclohexyl-22,23-dihydroavermectin B1 monosaccharide,
5-(N-(3-pyridylcarbonyl)-carbamoyloximino)-25-cyclohexyl-22,23-dihydroavermectin B1 monosaccharide,
5-(N-(3-pyridyl)-carbamoyloximino)-25-cyclohexyl-22,23-dihydroavermectin B1 monosaccharide,
5-(N-allylcarbamoyloximino)-25-cyclohexyl-22,23-dihydroavermectin B1 monosaccharide.

8. A pharmaceutical or veterinary composition, comprising a compound according to any preceding claim and a pharmaceutically acceptable carrier or excipient.

9. A compound according to any one of claims 1 to 7 for use in animal or human medicine.

10. A compound according to any one of claims 1 to 7, for use as an antiparasitic agent.

11. Use of a compound according to any one of claims 1 to 7 for making a medicament for treatment or prophylaxis of flea infestations.

12. A process for preparing a compound of formula (I) as claimed in claim 1 wherein R¹, R², R³, R⁴ and R⁵ are as previously defined which comprises the steps (1) of oxidising a compound of formula (II): 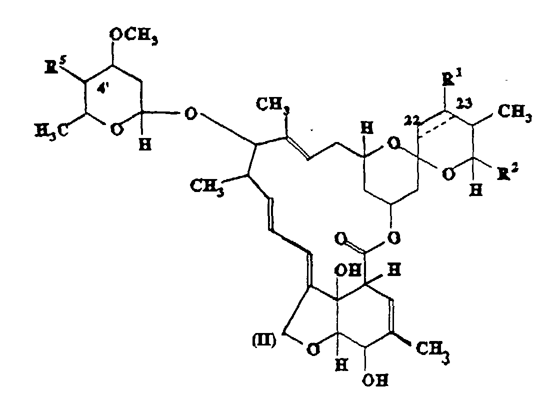 wherein the broken line, R¹ and R², are as defined above and R⁵ is OH or R⁵ is L-α-oleandrosyloxy to yield a compound of formula (III): 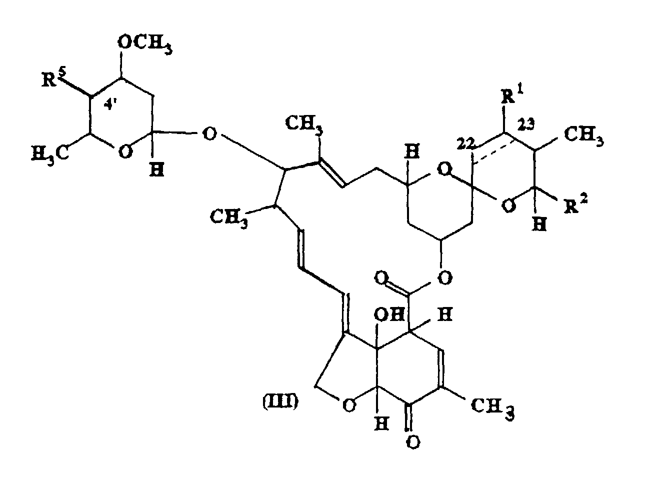
and (ii) allowing the compound of formula (III) to react with a compound of formula R⁴-O-NH₂ where R⁴ is as defined Above
and where R⁵ is α-oleandrosyloxy, hydrolysing the compound obtained to yield a compound of formula (I),
and (iii) if necessary replacing group R⁴ when the latter is H with said group capable of being hydrolysed in vivo to yield a compound in which R⁴ is H, and
if necessary,
(iv) hydrogenating the compound to reduce a double bond at the 22-23 position to a single bond.

## Patentansprüche

1. Verbindung der Formel (I) 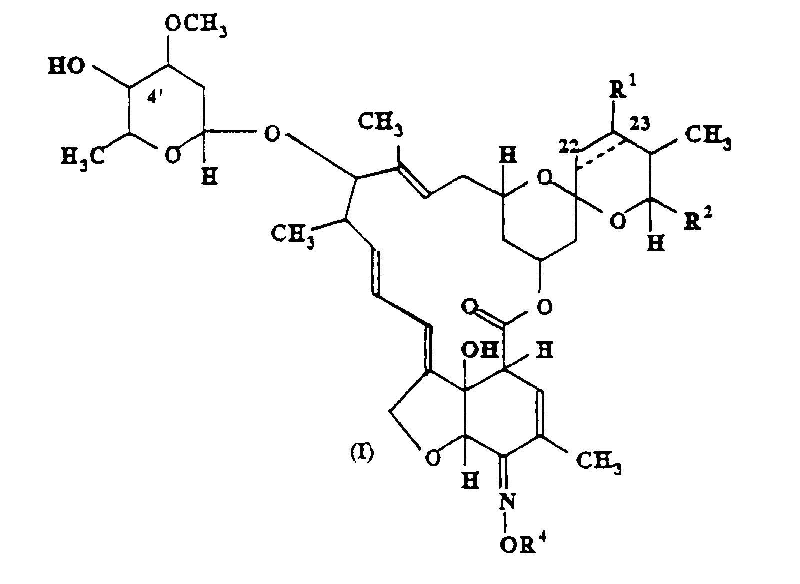 worin die Strichlinie in der 22-23-Stellung eine mögliche Bindung wiedergibt und entweder diese Bindung vorliegt und R¹ nicht vorliegt oder diese Bindung nicht vorliegt und R¹ H oder OH darstellt; R² eine C₁-C₈-Alkyl- oder C₃-C₈-Cycloalkylgruppe darstellt; und R⁴ H oder eine Gruppe darstellt, die in vivo unter Gewinnung einer Verbindung, in der R⁴ H darstellt, hydrolysiert werden kann.

2. Verbindung nach Anspruch 1, worin die Gruppe, die in vivo hydrolysiert werden kann, eine Acetyl-, t-Butylcarbonyl-, t-Butyloxycarbonyl-, Benzoyl-, Methylpiperazincarbonyl-, N-Methylcarbamoyl-, N,N-Dimethylcarbamoyl-, N-4-(Formylphenyl)carbamoyl-, N-(4-Diethylaminomethylphenyl)-carbonyl-, N-(4-Methyl-1-piperazinmethylphenyl)-carbonyl-, N-(3-Pyridylcarbonyl)-carbamoyl-, N-(3-Pyridyl)-carbamoyl- oder N-Allylcarbamoyl-Gruppe darstellt.

3. Verbindung nach Anspruch 1, worin R² Cyclohexyl darstellt.

4. Verbindung nach einem vorangehenden Anspruch, worin die mögliche Bindung in der 22-23-Stellung nicht vorliegt und R¹ H darstellt.

5. Eine der nachstehenden Verbindungen:
5-Oximino-22,23-dihydroavermectin B1a-Monosaccharid,
5-Oximino-22,23-dihydro-25-cyclohexylavermectin B1-Monosaccharid,
5-Oximino-25-cyclohexylavermectin B2-Monosaccharid,
5-Oximino-25-cyclohexylavermectin B1-Monosaccharid,
5-Oximinoavermectin B1a-Monosaccharid.

6. Verbindung 5-Oximino-22,23-dihydro-25-cyclohexylavermectin B1-Monosaccharid.

7. Verbindung nach Anspruch 1, worin die Gruppe R⁴ eine in vivo hydrolysierbare Gruppe darstellt und die Verbindung ist:
5-(Trimethylacetyloximino)-25-cyclohexyl-22,23-dihydroavermectin B1-Monosaccharid,
5-(Benzoyloximino)-25-cyclohexyl-22,23-dihydroavermectin B1-Monosaccharid,
5-(N-Methylcarbamoyloximino)-25-cyclohexyl-22,23-dihydroavermectin B1-Monosaccharid,
5-(N,N-Dimethylcarbamoyloximino)-25-cyclohexyl-22,23-dihydroavermectin B1-Monosaccharid,
5-(4-Methylpiperazinyl-1-carbonyloximino)-25-cyclohexyl-22,23-dihydroavermectin B1-Monosaccharid,
5-(t-Butyloxycarbonyloximino)-25-cyclohexyl-22,23-dihydroavermectin B1-Monosaccharid,
5-(N-(4-Formylphenyl)-carbamoyloximino)-25-cyclohexyl-22,23-dihydroavermectin B1-Monosaccharid,
5-(N-(4-(Diethylaminomethyl)-phenyl)-carbonyloximino)-25-cyclohexyl-22,23-dihydroavermectin B1-Monosaccharid,
5-(N-(4-(4-Methyl-1-piperazinylmethyl)phenyl)-carbonyloximino)-25-cyclohexyl-22,23-dihydroavermectin B1-Monosaccharid,
5-(N-(3-Pyridylcarbonyl)-carbamoyloximino)-25-cyclohexyl-22,23-dihydroavermectin B1-Monosaccharid,
5-(N-(3-Pyridyl)-carbamoyloximino)-25-cyclohexyl-22,23-dihydroavermectin B1-Monosaccharid,
5-(N-Allylcarbamoyloximino)-25-cyclohexyl-22,23-dihydroavermectin B1-Monosaccharid.

8. Pharmazeutische oder veterinäre Zusammensetzung, umfassend eine Verbindung nach einem vorangehenden Anspruch und einen pharmazeutisch verträglichen Träger oder Exzipienten.

9. Verbindung nach einem der Ansprüche 1 bis 7 zur Verwendung als Arzneimittel in der Veterinär- oder Humanmedizin.

10. Verbindung nach einem der Ansprüche 1 bis 7 zur Verwendung als antiparasitäres Mittel.

11. Verwendung einer Verbindung nach einem der Ansprüche 1 bis 7 zur Herstellung eines Arzneimittels zur Behandlung oder Prophylaxe von Flohbefall.

12. Verfahren zur Herstellung einer Verbindung der Formel (I) nach Anspruch 1, worin R¹, R², R³, R⁴ und R⁵ wie vorstehend definiert sind, das die Schritte umfaßt (1) Oxidieren einer Verbindung der Formel (II): 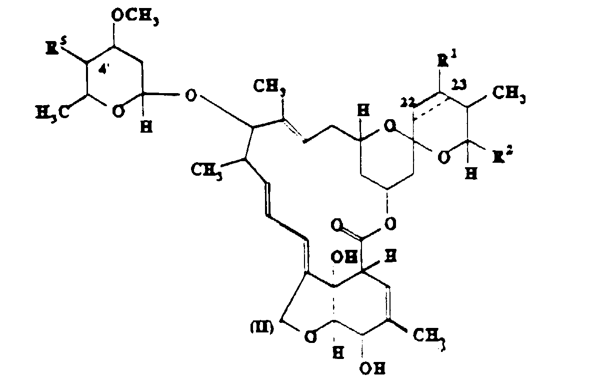 worin die Strichlinie, R¹ und R² wie vorstehend definiert sind und R⁵ OH darstellt oder R⁵ L-α-Oleandrosyloxy darstellt unter Gewinnung einer Verbindung der Formel (III): 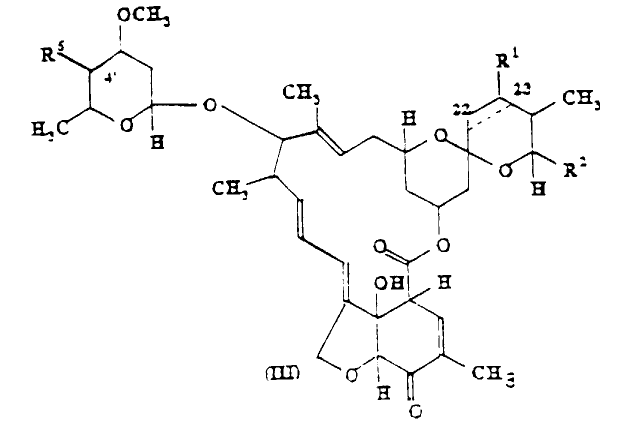
und (ii) Umsetzen der Verbindung der Formel (III) mit einer Verbindung der Formel R⁴-O-NH₂, worin R⁴ wie vorstehend definiert ist, und, wenn R⁵ α-Oleandrosyloxy darstellt, Hydrolysieren der erhaltenen Verbindung unter Gewinnung einer Verbindung der Formel (I),
und (iii), falls erforderlich, Austauschen der Gruppe R⁴, wenn die letztere H darstellt, mit der Gruppe, die in vivo hydrolysiert werden kann, unter Gewinnung einer Verbindung, worin R⁴ H darstellt, und, falls erforderlich,
(iv) Hydrieren der Verbindung, um eine Doppelbindung in der 22-23-Stellung zu einer Einfachbindung zu reduzieren.

## Revendications

1. Composé de formule (I) : 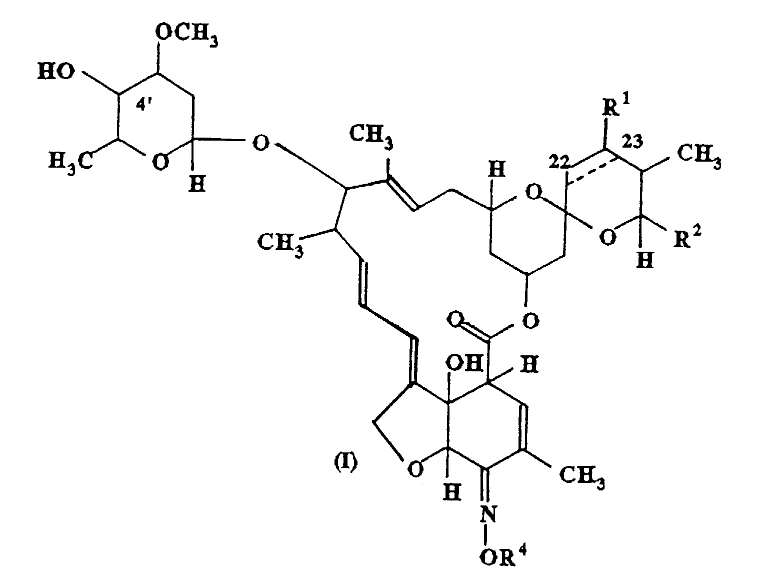 dans laquelle la ligne discontinue en position 22-23 représente une liaison facultative et cette liaison est présente et R¹ est absent ou bien cette liaison est absente et R¹ représente H ou un groupe OH ; R² représente un groupe alkyle en C₁ à C₈ ou un groupe cycloalkyle en C₃ à C₈ ; et R⁴ représente H ou un groupe pouvant être hydrolysé in vivo en donnant un composé dans lequel R⁴ représente H.

2. Composé suivant la revendication 1, dans lequel le groupe pouvant être hydrolysé in vivo est un groupe acétyle, tertio-butylcarbonyle, tertio-butyloxycarbonyle, benzoyle, méthylpipérazinecarbonyle, N-méthylcarbamoyle, N,N-diméthylcarbamoyle, N-4-(formylphényl)carbamoyle, N-(4-diéthylaminométhylphényl)-carbonyle, N-(4-méthyl-1-pipérazine-méthylphényl)-carbonyle, N-(3-pyridylcarbonyl)-carbamoyle, N-(3-pyridyl)-carbamoyle ou N-allylcarbamoyle.

3. Composé suivant la revendication 1, dans lequel R² représente un groupe cyclohexyle.

4. Composé suivant l'une quelconque des revendications précédentes, dans lequel la liaison facultative en position 22-23 est absente et R¹ représente H.

5. Un quelconque des composés suivant :
monosaccharide de 5-oximino-22,23-dihydroavermectine B1a,
monosaccharide de 5-oximino-22,23-dihydro-25-cyclohexylavermectine B1,
monosaccharide de 5-oximino-25-cyclohexylavermectine B2,
monosaccharide de 5-oximino-25-cyclohexylavermectine B1,
monosaccharide de 5-oximino-avermectine B1a.

6. Composé consistant en le monosaccharide de 5-oximino-22,23-dihydro-25-cyclohexylavermectine B1.

7. Composé suivant la revendication 1, dans lequel le groupe R⁴ est un groupe hydrolysable in vivo et ledit composé consiste en :
le monosaccharide de 5- (triméthylacétyloximino) - 25-cyclohexyl-22,23-dihydro-avermectine B1,
le monosaccharide de 5-(benzoyloximino)-25-cyclohexyl-22,23-dihydro-avermectine B1,
le monosaccharide de 5-(N-méthylcarbamoyloximino)-25-cyclohexyl-22,23-dihydro-avermectine B1,
le monosaccharide de 5-(N,N-diméthylcarbamoyloximino)-25-cyclohexyl-22,23-dihydro-avermectine B1,
le monosaccharide de 5-(4-méthylpipérazinyl-1-carbonyloximino)-25-cyclohexyl-22,23-dihydro-avermectine B1,
le monosaccharide de 5- (tertio-butyloxycarbonyloximino)-25-cyclohexyl-22,23-dihydro-avermectine B1,
le monosaccharide de 5-(N-(4-formylphényl)-carbamoyloximino)-25-cyclohexyl-22,23-dihydro-avermectine Bl,
le monosaccharide de 5-(N-(4-(diéthylaminométhyl)phényl)-carbonyloximino)-25-cyclohexyl-22,23-dihydroavermectine B1,
le monosaccharide de 5-(N-(4-(4-méthyl-1-pipérazinylméthyl)phényl)-carbonyloximino)-25-cyclohexyl-22,23-dihydro-avermectine B1,
le monosaccharide de 5-(N-(3-pyridylcarbonyl)-carbamoyloximino)-25-cyclohexyl-22,23-dihydro-avermectine B1,
le monosaccharide de 5-(N-(3-pyridyl)-carbamoyloximino)-25-cyclohexyl-22,23-dihydro-avermectine B1,
le monosaccharide de 5-(N-allylcarbamoyloximino)-25-cyclohexyl-22,23-dihydro-avermectine B1.

8. Composition pharmaceutique ou vétérinaire, comprenant un composé suivant l'une quelconque des revendications précédentes et un support ou excipient pharmaceutiquement acceptable.

9. Composé suivant l'une quelconque des revendications 1 à 7, destiné à être utilisé en médecine vétérinaire ou médecine humaine.

10. Composé suivant l'une quelconque des revendications 1 à 7, destiné à être utilisé comme agent antiparasitaire.

11. Utilisation d'un composé suivant l'une quelconque des revendications 1 à 7 pour la préparation d'un médicament destiné au traitement ou à la prophylaxie d'infestations par des puces.

12. Procédé pour la préparation d'un composé de formule (I) suivant la revendication 1, dans lequel R¹, R², R³, R⁴ et R⁵ répondent aux définitions précitées, qui comprend les étapes consistant (i) à oxyder un composé de formule (II) : 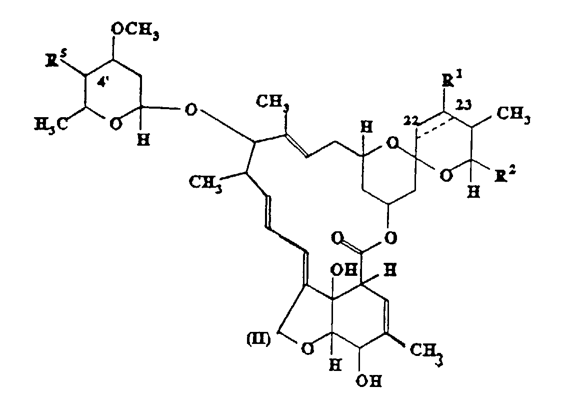 dans laquelle la ligne discontinue, R¹ et R² répondent aux définitions précitées et R⁵ représente un groupe OH ou R⁵ représente un groupe L-α-oléandrosyloxy, ce qui donne un composé de formule (III) : 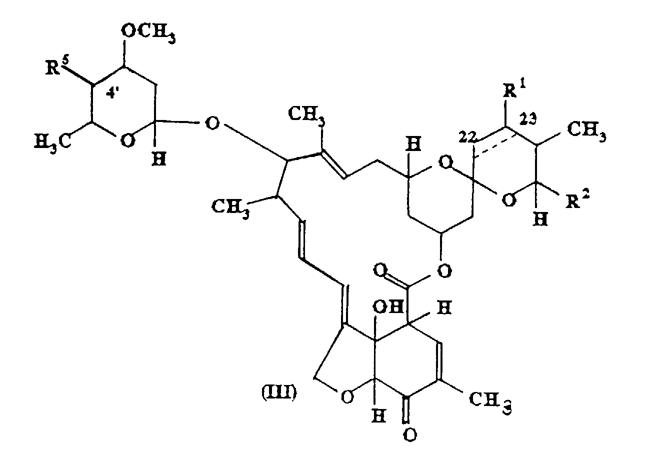
et (ii) à laisser le composé de formule (III) réagir avec un composé de formule R⁴-O-NH₂ dans laquelle R⁴ répond à la définition précitée,
et lorsque R⁵ représente un groupe α-oléandrosyloxy, à hydrolyser le composé obtenu, ce qui donne un composé de formule (I),
et (iii) si nécessaire, à remplacer le groupe R⁴ lorsque ce dernier représente H par ledit groupe pouvant être hydrolysé in vivo, ce qui donne le composé dans lequel R⁴ représente H, et
si nécessaire,
(iv) à hydrogéner le composé pour réduire une double liaison en position 22-23 en une liaison simple.
